(19) Europäisches Patentamt
European Patent Office
Office européen des brevets

(11) **EP 3 592 209 B1**

(12) **EUROPEAN PATENT SPECIFICATION**

(45) Date of publication and mention
of the grant of the patent:
**16.09.2020 Bulletin 2020/38**

(51) Int Cl.:
***A61B 5/00*** *(2006.01)*    ***A61B 5/0215*** *(2006.01)*
***A61B 5/053*** *(2006.01)*

(21) Application number: **18708953.7**

(22) Date of filing: **27.02.2018**

(86) International application number:
**PCT/EP2018/054824**

(87) International publication number:
**WO 2018/162285 (13.09.2018 Gazette 2018/37)**

(54) **MEASURING A PROPERTY IN A BODY**

MESSUNG EINER EIGENSCHAFT IN EINEM KÖRPER

MESURE D'UNE PROPRIÉTÉ DANS UN CORPS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.03.2017 EP 17160133**
**26.05.2017 EP 17173013**

(43) Date of publication of application:
**15.01.2020 Bulletin 2020/03**

(73) Proprietor: **Koninklijke Philips N.V.**
**5656 AG Eindhoven (NL)**

(72) Inventors:
• **DOODEMAN, Gerardus, Johannes, Nicolaas**
**5656 AE Eindhoven (NL)**
• **DE WIJS, Willem-Jan, Arend**
**5656 AE Eindhoven (NL)**

• **KAHLERT, Joachim**
**5656 AE Eindhoven (NL)**

(74) Representative: **Philips Intellectual Property & Standards**
**High Tech Campus 5**
**5656 AE Eindhoven (NL)**

(56) References cited:
**WO-A2-2004/014456     US-A1- 2003 037 591**
**US-A1- 2010 277 175**

• **VILLE VIIKARI ET AL: "RFID MEMS Sensor Concept Based on Intermodulation Distortion", IEEE SENSORS JOURNAL, IEEE SERVICE CENTER, NEW YORK, NY, US, vol. 9, no. 12, 1 December 2009 (2009-12-01), pages 1918-1923, XP011278923, ISSN: 1530-437X, DOI: 10.1109/JSEN.2009.2031809**

EP 3 592 209 B1

## Description

TECHNICAL FIELD OF THE INVENTION

The invention relates to a measurement circuit, measurement system, and measurement method for measuring a property in a body.

BACKGROUND OF THE INVENTION

Pressure measurements inside the body of a human or other animal patient can be used to assess a multitude of physical ailments, such as coronary artery stenosis, liver portal vein pressure, and pulmonary vein pressure, to mention just a few. In order to be able to measure pressure, in many cases at least two wires must be integrated into a device that can be positioned inside the body, which puts conditions on the interconnection of the device outside the body.

In patent applications WO2012/109039, WO2014/164734 and WO2015/099845 a pressure sensitive resonant circuit is described. The resonant frequency of the circuit depends on pressure because it includes a pressure dependent inductance and/or capacitance. The resonant circuit is connected at one side to the human body, and on the other side to a guide wire. The resonant frequency is determined by measuring the impedance of the resonant circuit via the guidewire and the return loop of the body.

One disadvantage of this measurement method is that the signal path through the body and the guide wire are part of the resonant circuit. This means that the behavior of the resonant circuit changes with all changes in orientation of the body and guide wire and changes with drift and environmental alterations

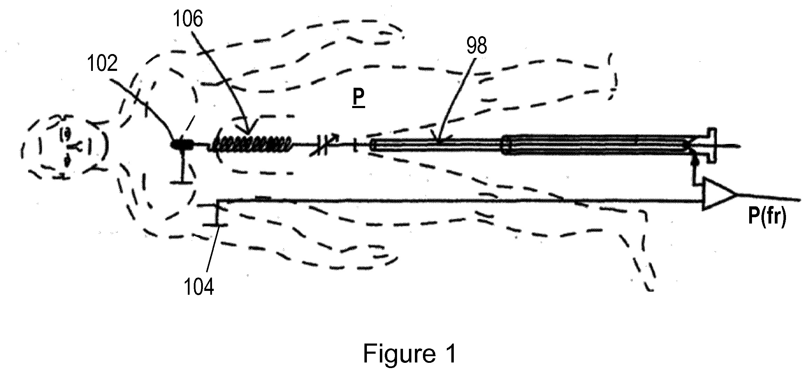

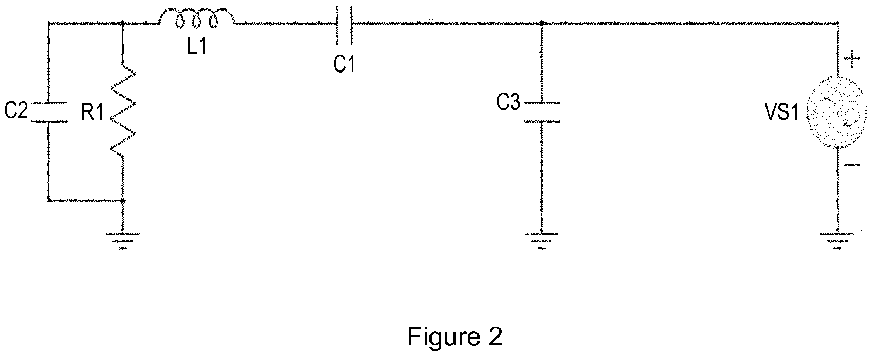

Figure 1 shows a measurement system according to patent application WO2014/164734. An inductance is connected in series with a pressure dependent capacitor to form a resonance circuit 106, and are connected to an electrode 102 and a guide wire 98 to form a circuit which is electrically equivalent to the electrical circuit shown in Figure 2. In Figure 2, C1 is the pressure dependent capacitor, LI is the inductance in the resonant circuit 106, R1 and C2 represent the body resistance and capacitance respectively, and C3 represents the parasitic capacitance of the guide wire 98.

The resonance circuit 106 is inserted into the body of the patient P, who is connected to ground potential through a ground electrode 104.

The voltage source VS1 generates a swept frequency signal. The current delivered by the voltage source is measured and the voltage to current ratio is a measure of the impedance of the circuit as function of frequency. The resonance frequency changes as a function of pressure and the resonance frequency will also change when any parasitic element in the body return path is altered e.g. by body movement, temperature changes or drift of component properties.

Document WO 2004/014456 discloses a flexible sensor for wirelessly determining a physical property in a patient's artery. The sensor comprises a self-contained resonant circuit which is variable in response to a physical property of a patient. The sensor features an inductive-capacitive (LC) resonant circuit with a variable capacitor. The LC circuit may further comprise a non-linear element, such as a diode. The presence of the non-linear element in various configurations within the LC circuit can be used to modulate an incoming signal from a receiving device and produce different harmonics of the original signal. The read-out circuitry can be tuned to receive the particular harmonic frequency that is produced and use this signal to reconstruct the fundamental frequency of the sensor. As the incoming signal and the return signal are at different frequencies, the incoming signal can be transmitted continuously, and the return signal can also be received continuously. However, WO 2004/014456 does not provide any hints to exploit amplitude ratios of odd order intermodulation products in order to extract measured body properties.

SUMMARY OF THE INVENTION

It is an object of the present invention to provide an improved measurement circuit, measurement system and measurement method for measuring a property in a body.

The invention provides a measurement circuit, measurement system and measurement method for measuring a property in a body to mitigate the problems in the prior art. The invention is defined by appended claims 1-14.

In a first aspect of the present invention, there is provided a measurement system for measuring a property in a body, the measurement system comprising:

a measurement circuit for measuring a property in a body, the measurement circuit comprising:

a resonant circuit comprising a first element and a second element, wherein at least one of the first element and the second element is configured such that a property thereof is dependent on the property in the body; and a non-linear element arranged to generate intermodulation products when the circuit is driven by an input signal comprising a first component at a first frequency and a second component at a second frequency;

a driving circuit for providing a driving signal to the measurement circuit, wherein the driving signal comprises a first component at a first frequency and a second component at a second frequency; and an analyzer circuit for analyzing a response of the measurement circuit in order to obtain a measurement of the property, wherein the analyzer circuit is configured to measure a ratio of the amplitudes of odd order intermodulation products resulting from the first and second components of the driving signal.

In some embodiments, at least one of the first element and the second element is configured such that an electrical property thereof is dependent on the property in the body.

In some embodiments, the first element is an inductance and the second element is a capacitor.

The first element and the second element may be arranged in parallel.

The non-linear element may comprise two antiparallel diodes.

In some embodiments, the first element is an inductance and the second element is a capacitor, the inductance and the capacitor are arranged in parallel, and the two antiparallel diodes are connected in parallel with the inductance and the capacitor.

In some embodiments, an electrical property of at least one of the first element and the second element is dependent on pressure in the body.

The driving circuit may be arranged to provide the driving signal comprising the first component at the first frequency and the second component at the second frequency, and wherein the first frequency is above a resonant frequency of the resonant circuit and the second frequency is below the resonant frequency of the resonant circuit.

The driving circuit may be arranged to provide the driving signal comprising the first component at the first frequency and the second component at the second frequency, wherein the first and the second frequency fall within the bandwidth of the resonant circuit.

In some embodiments, the analyzer circuit comprises a frequency analyzer.

The measurement system may further comprise a wire connection connecting the driving circuit and the measurement circuit.

In other embodiments, the measurement system may further comprise a transmitter arranged to wirelessly transmit the driving signal from the driving circuit to the measurement circuit.

In some embodiments, the system is arranged to wirelessly transmit the response of the measurement circuit to the analyzer circuit.

In a second aspect of the present invention, there is provided a method of measuring a property in a body, the method comprising:

applying a driving signal to a measurement circuit, wherein the driving signal comprises a first component at a first frequency and a second component at a second frequency, and wherein the measurement circuit comprises:

a resonant circuit comprising a first element and a second element, wherein at least one of the first element and the second element is configured such that a property thereof is dependent on the property in the body; and
a non-linear element arranged to generate intermodulation products when the circuit is driven by an input signal comprising a first component at a first frequency and a second component at a second frequency; and

analyzing a response of the measurement circuit in order to obtain a measurement of the property, wherein analyzing the response of the measurement circuit comprises measuring a ratio of the amplitudes of odd order intermodulation products resulting from the first and second components of the driving signal.

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a measurement system according to the prior art;
Figure 2 shows an equivalent schematic circuit diagram of the measurement system of Figure 1;
Figure 3 illustrates an embodiment of the measurement system according to the invention;
Figure 4 illustrates a further embodiment of the measurement system according to the invention;
Figure 5 shows signals generated in an embodiment of the measurement system according to the invention;
Figure 6 shows further signals generated in an embodiment of the measurement system according to the invention;
Figure 7 illustrates an effect of a shift in resonant frequency in an embodiment of the measurement system according to the invention; and
Figure 8 is a flow chart illustrating a method according to an aspect of the invention.

DETAILED DESCRIPTION OF PREFERRED EMBODIMENTS

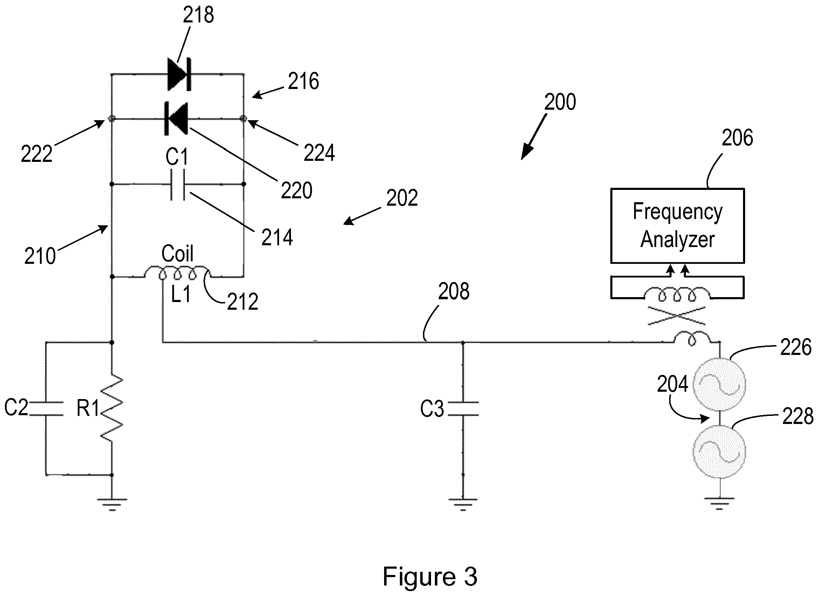

Figure 3 illustrates a first embodiment of a measurement system in accordance with the invention.

The measurement system 200 shown in Figure 3 comprises a measurement circuit 202, a driving circuit 204 for providing a driving signal to the measurement circuit 202, and a frequency analyzer circuit 206.

The measurement circuit 202 is connected to a guide wire 208, which connects the measurement circuit 202 to the driving circuit 204 and the frequency analyzer circuit 206, and is also used to guide the measurement circuit 202 to the intended operating location in the body of a human or animal patient.

The measurement circuit 202 includes a resonant circuit 210, which comprises a coil 212 having an inductance L1 and a capacitor 214 having a capacitance C1. In this illustrated embodiment, the inductor 212 and the capacitor 214 are connected in parallel. In other embodiments, an inductor and a capacitor are connected in series. At least one of the inductor 212 and the capacitor 214 is configured such that an electrical property thereof is dependent on a property within the body that is intended to be measured.

In one embodiment, the capacitor 214 is configured such that its capacitance is dependent on a pressure at the operating location within the body. For example, the capacitor 214 may contain a flexible, impressionable dielectric layer.

In other embodiments, the resonant circuit 210 includes an inductor whose inductance is dependent on the pressure. For example, the inductor may be bendable, or may include an impressionable coil, or a coil with a pressure-sensitive movable conductive or magnetic core.

In other embodiments, at least one of the inductor and the capacitor is configured such that an electrical property thereof is dependent on a different property within the body, for example such as the permittivity or permeability of bodily fluids such as blood, allowing variations in one of these properties to be measured.

The measurement circuit 202 further includes a non-linear element 216. In this illustrated embodiment, the non-linear element 216 comprises first and second diodes 218, 222 connected in anti-parallel. That is, current can flow from the node 222 to the node 224 in the forward direction of the first diode 218, and can flow from the node 224 to the node 222 in the forward direction of the second diode 220.

The electrical circuits can be made according to methods described in International Patent Applications WO2017/013224 and WO2016/207041, where transducer circuits are constructed using pressure electrical contacts and isolating adhesive layers. These methods, and in particular, the methods of integrating and interconnecting electrical elements (transducers) to wiring in a form factor suitable for guide wires, can also be used to construct pressure dependent capacitors.

Alternatively, the transducer may be replaced by an air capacitor and thin diodes (<150 μm thick, preferably <50μm thick, and most preferably <15μm thick), and the wires used for interconnection can form the coil structure and potentially an antenna structure, all in a form factor of <200μm, preferably <100μm, and most preferably <50μm thick, with the consequence that integration in a guide wire or implantable device is feasible.

Figure 3 also shows the electrical effect of the measurement circuit 202, in use. Specifically, Figure 3 shows that, when the measurement circuit 202 is at its intended operating location in the body of a human or animal patient, the body has an effective resistance R1 and an effective capacitance C2. The guide wire 208 also has a parasitic capacitance C3. The effective resistance R1 and the effective capacitance C2 of the body are sensitive to movement of the body, amongst other things.

Therefore, in the embodiment shown in Figure 3, the resonant circuit 210 and the non-linear element 216 are isolated from the body resistance R1 and capacitance C2, in order to reduce the effect of any changes in the body resistance R1 and capacitance C2 on the resonant frequency of the resonant circuit 210.

Specifically, the guide wire 208 is connected to the resonant circuit 210 at a tap of the coil 212. This provides a good degree of isolation of the variable guide wire impedance from the resonant circuit.

The driving circuit 204 is configured to generate a driving signal comprising a first component at a first frequency and a second component at a second frequency. This is represented in Figure 3 by a first driving signal source 226 and a second driving signal source 228.

The operation of the driving circuit 204 is described in more detail below.

The frequency analyzer circuit 206 detects the response of the measurement circuit 202 to the driving signal.

The operation of the frequency analyzer circuit 206 is described in more detail below.

A resistance can be included in the guide wire 208 or in the measurement circuit 210 where it connects to the guide wire, in order to prevent excessive current being driven into the body.

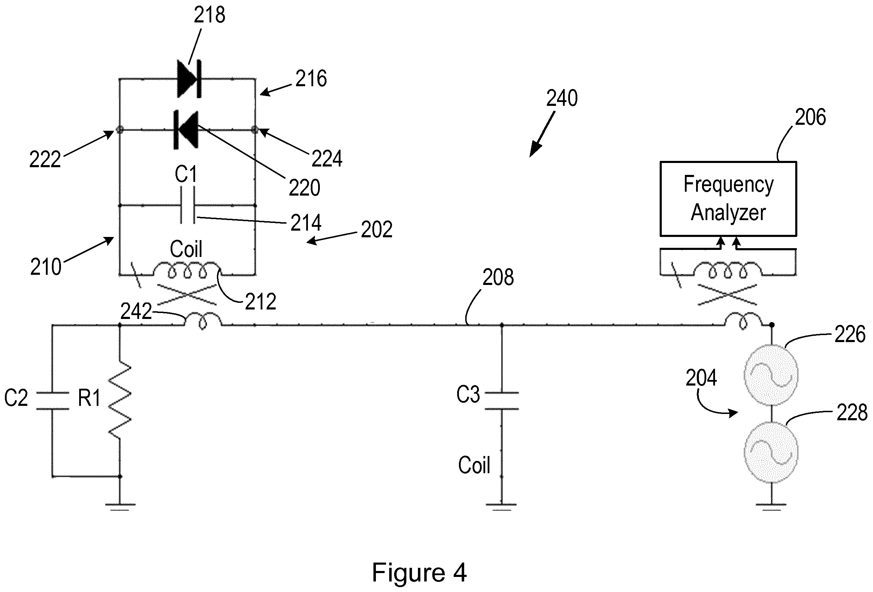

Figure 4 illustrates a second embodiment of a measurement system in accordance with the invention.

The measurement system 240 shown in Figure 4 is similar in some respects to the measurement system 200 shown in Figure 3, and the same reference numerals are used to indicate features that are the same. Thus, the measurement system 240 shown in Figure 4 comprises a measurement circuit 202, a driving circuit 204 for providing a driving signal to the measurement circuit 202, and a frequency analyzer circuit 206.

The measurement circuit 202 is connected to a guide wire 208, which connects the measurement circuit 202 to the driving circuit 204 and the frequency analyzer circuit 206, and is also used to guide the measurement circuit 202 to the intended operating location in the body of a human or animal patient.

The measurement circuit 202 includes a resonant circuit 210, which comprises a coil 212 having an inductance LI and a capacitor 214 having a capacitance C1. In this illustrated embodiment, the inductor 212 and the capacitor 214 are connected in parallel. In other embodiments, an inductor and a capacitor are connected in series. At least one of the inductor 212 and the capacitor 214 is configured such that an electrical property thereof is dependent on a property within the body that is intended to be measured.

In one embodiment, the capacitor 214 is configured such that its capacitance is dependent on a pressure at the operating location within the body. For example, the capacitor 214 may contain a flexible, impressionable dielectric layer.

In other embodiments, the resonant circuit 210 includes an inductor whose inductance is dependent on the pressure. For example, the inductor may be bendable, or may include an impressionable coil, or a coil with a pressure-sensitive movable conductive or magnetic core.

In other embodiments, at least one of the inductor and the capacitor is configured such that an electrical property thereof is dependent on a different property within the body, for example such as the permittivity or permeability of bodily fluids such as blood, allowing variations in one of these properties to be measured.

The measurement circuit 202 further includes a non-linear element 216. In this illustrated embodiment, the non-linear element 216 comprises first and second diodes 218, 222 connected in anti-parallel. That is, current can flow from the node 222 to the node 224 in the forward direction of the first diode 218, and can flow from the node 224 to the node 222 in the forward direction of the second diode 220.

The electrical circuits can be made according to methods described in International Patent Applications WO2017/013224 and WO2016/207041, where transducer circuits are constructed using pressure electrical contacts and isolating adhesive layers. These methods, and in particular, the methods of integrating and interconnecting electrical elements (transducers) to wiring in a form factor suitable for guide wires, can also be used to construct pressure dependent capacitors.

Alternatively, the transducer may be replaced by an air capacitor and thin diodes (<150 $\mu$m thick, preferably <50$\mu$m thick, and most preferably <15$\mu$m thick), and the wires used for interconnection can form the coil structure and potentially an antenna structure, all in a form factor of <200$\mu$m, preferably <100$\mu$m, and most preferably <50$\mu$m thick, with the consequence that integration in a guide wire or implantable device is feasible.

Figure 4 also shows the electrical effect of the measurement circuit 202, in use. Specifically, Figure 4 shows that, when the measurement circuit 202 is at its intended operating location in the body of a human or animal patient, the body has an effective resistance R1 and an effective capacitance C2. The guide wire 208 also has a parasitic capacitance C3. The effective resistance R1 and the effective capacitance C2 of the body are sensitive to movement of the body, amongst other things.

Therefore, in the embodiment shown in Figure 4, the resonant circuit 210 and the non-linear element 216 are isolated from the body resistance R1 and capacitance C2, in order to reduce the effect of any changes in the body resistance R1 and capacitance C2 on the resonant frequency of the resonant circuit 210.

Specifically, the guide wire 208 includes an inductor 242, which has an inductive connection to the coil 212. Because of the very low currents flowing through the guide wire, due to the high resistance R1 and low capacitance C2, the mutual inductance will cause minimal effects on the resonant frequency of the resonant circuit.

The driving circuit 204 is configured to generate a driving signal comprising a first component at a first frequency and a second component at a second frequency. This is represented in Figure 3 by a first driving signal source 226 and a second driving signal source 228.

The operation of the driving circuit 204 is described in more detail below.

The frequency analyzer circuit 206 detects the response of the measurement circuit 202 to the driving signal.

The operation of the frequency analyzer circuit 206 is described in more detail below.

A resistance can be included in the guide wire 208 or in the measurement circuit 210 where it connects to the guide wire, in order to prevent excessive current being driven into the body.

Thus, Figures 3 and 4 shows embodiments in which the driving signal is applied in a wired manner to the resonance circuit, and the response of the resonance circuit is returned in a wired manner to the frequency analyzer.

In other embodiments, the driving signal is applied wirelessly to the resonance circuit and/or the response is returned wirelessly to the frequency analyzer. For example, the driving signal can be transmitted to the resonance circuit via an electrical conductor in a guidewire, with the return path for the response being provided by the patient body. The contact between the guide wire and the measurement circuit may be made by means of a brush contact (as the exact value of the contact resistance is not critical). For a body return path, a standard electrocardiogram (ECG) lead can be connected to the system (or patched through).

In some embodiments, the driving signal comprising the two frequencies can also be sent wirelessly through the body to the guide wire, and the intermodulation products sent back also via the guide wire, using it as an antenna, with suitable choice of driving and transmission frequencies.

In other embodiments, the two frequency components of the driving signal can be transmitted using ultrasonic transducers (as described in US 2013/0060139 A1) and converted to electrical signals using for example a polyvinylidene fluoride (pvdf) transducer or a piezoelectric transducer (PZT) to avoid having to also receive reflected ultrasonic energy, but to receive the transduced electrical energy through the guide wire acting as antenna.

Similarly, in other embodiments, a similar system can be constructed using optical frequencies and photo-diodes.

The embodiments shown in Figures 3 and 4, and the further embodiments described above, operate in generally the same way. As mentioned above, the driving circuit 204 is configured to generate a driving signal comprising a first component at a first frequency and a second component at a second frequency. The first and second components are preferably sinusoidal signals. The use of the two sinusoidal signals at discrete frequencies avoids the need to sweep through a frequency range of interest whenever a measurement is required.

As described in more detail below, in some embodiments, the first and second frequencies are set up so that the baseline resonant frequency of the resonant circuit 210 is midway between them. Thus, to set this up, the resonant circuit is first excited by a frequency scan across the expected range of the resonant frequency. This range should be relatively narrow, and so the frequency scan will not need to cover a wide range of frequencies. This allows the baseline resonant frequency to be determined. Then the first frequency and the second frequency to be generated by the driving circuit in use are adjusted so that the baseline resonance frequency is midway between them.

The baseline resonant frequency, and the first frequency and the second frequency, may be at radio frequencies, for example between 50MHz and 2GHz.

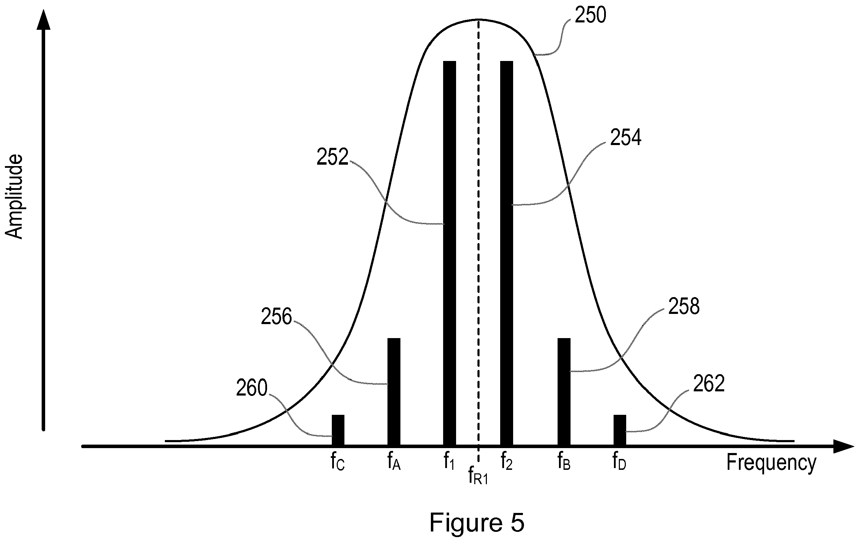

Figure 5 shows the effect of this operation, in a situation where the resonance curve of the resonant circuit 210 is illustrated by the line 250. The resonant circuit 210 has a baseline resonant frequency, for example at a time before the measurement circuit is inserted into the body of the patient, of $f_{R1}$. When the resonant circuit 210 is excited by a signal at this resonant frequency $f_{R1}$, it produces a response having the maximum power. When the resonant circuit 210 is excited by a signal at a different frequency, it produces a response having a lower power, which is determined by the shape of the resonance curve 250.

The resonant frequency $f_{R1}$ of the measurement circuit 202 depends on the inductance LI of the inductor 212 and the capacitance C1 of the capacitor 214. Specifically:

$$f_{R1} = \frac{1}{2\pi \sqrt{L1 \bullet C1}} .$$

For an example, if the inductance LI of the inductor 212 is equal to 20nH, and the capacitance C1 of the capacitor 214 is equal to 5pF, then the resonant frequency $f_{R1}$ of the measurement circuit 202 is approximately 500MHz.

Specifically, Figure 5 shows the situation when the driving circuit 204 is configured to generate a driving signal comprising a first component at a first frequency $f_1$ and a second component at a second frequency $f_2$. The first frequency $f_1$ and the second frequency $f_2$ are chosen such that the baseline resonant frequency $f_{R1}$ is between the first frequency $f_1$ and the second frequency $f_2$.

The first frequency $f_1$ and the second frequency $f_2$ are chosen in this example such that they differ by less than 10%. More specifically, the first frequency $f_1$ and the second frequency $f_2$ are chosen in this example such that they differ by less than 1%. More specifically still, the first frequency $f_1$ and the second frequency $f_2$ are chosen in this example such that they differ by less than 0.1%.

As an illustrative example, if the resonant frequency $f_{R1}$ of the measurement circuit 202 is approximately 500 MHz, the first frequency $f_1$ and the second frequency $f_2$ may be chosen such that they differ by 100 kHz, which is 0.02%.

The quality factor (Q factor) of the resonant circuit 210 depends on the effective resistance of the resonant circuit. This effective resistance is reduced in the embodiments shown in Figures 3 and 4, in which the resonant circuit 210 is isolated from the parasitic resistance of the body. Reducing the effective resistance has the effect of increasing the Q factor of the resonant circuit 210 and thus reducing the effective bandwidth of the circuit, which may be defined as the frequency difference between the two frequencies at which the power of the response of the resonant circuit is one half of the peak power of the response at the resonant frequency.

The first frequency $f_1$ and the second frequency $f_2$ are chosen in this example such that they are both within the effective bandwidth of the resonant circuit 210.

Thus, Figure 5 shows that the measurement circuit 202 produces a response 252 to the first component of the driving signal at the first frequency $f_1$ and produces a response 254 to the second component of the driving signal at the second frequency $f_2$. Where the first frequency $f_1$ and the second frequency $f_2$ are chosen such that the baseline resonant frequency $f_{R1}$ is midway between the first frequency $f_1$ and the second frequency $f_2$, as shown by way of illustration in Figure 5, the response 252 and the response 254 are of similar magnitude.

The effect of including the non-linear element 216 in the measurement circuit 202 is that its response also includes harmonics and intermodulation components. By choosing the first and second frequencies to be close to the resonant frequency of the measurement circuit 202, the harmonics, at multiples of the first and second frequencies, and the even-order intermodulation components are effectively filtered out by the frequency-dependent measurement circuit 202, and so, in the bandwidth of interest, it is the odd-order intermodulation components that are relevant. The resonant circuit 210 also acts as a narrowband bandpass system that has the effect of suppressing out of band noise.

Figure 5 illustrates the third-order intermodulation components. Specifically, Figure 5 illustrates a first third-order intermodulation component 256 at the frequency $f_A$ (= $2.f_1 - f_2$) and a second third-order intermodulation component 258 at the frequency $f_B$ (= $2.f_2 - f_1$).

Since the baseline resonant frequency $f_{R1}$ is midway between the first frequency $f_1$ and the second frequency $f_2$, as shown by way of illustration in Figure 5, the first third-order intermodulation component 256 and the second third-order intermodulation component 258 are of similar magnitude.

Since the first frequency $f_1$ and the second frequency $f_2$ are close together, the frequency $f_A$ of the first third-order intermodulation component 256 and the frequency $f_B$ of the second third-order intermodulation component 258 are both within (or close to) the effective bandwidth of the resonant circuit 210.

Figure 5 also illustrates the fifth-order intermodulation components. Specifically, Figure 5 illustrates a first fifth-order intermodulation component 260 at the frequency $f_C$ (= $3.f_1 - 2.f_2$) and a second fifth-order intermodulation component 262 at the frequency $f_D$ (= $3.f_2 - 2.f_1$).

Since the baseline resonant frequency $f_{R1}$ is midway between the first frequency $f_1$ and the second frequency $f_2$, as shown by way of illustration in Figure 5, the first fifth-order intermodulation component 260 and the second fifth-order intermodulation component 262 are of similar magnitude.

If the first frequency $f_1$ and the second frequency $f_2$ are sufficiently close together, the frequency $f_C$ of the first fifth-order intermodulation component 260 and the frequency $f_D$ of the second fifth-order intermodulation component 262 are both within (or close to) the effective bandwidth of the resonant circuit 210.

When the measurement circuit 202 is inserted into the body of the patient, an electrical property of the resonant circuit may change. As described previously, the resonant circuit comprises a first element and a second element, and at least one of the first element and the second element is configured such that an electrical property thereof is dependent on a property in the body. In some embodiments, the resonant circuit comprises a capacitor and an inductor, and the capacitor is configured such that its capacitance is dependent on a pressure in the body.

If the capacitance changes due to the pressure in the body, the resonant frequency of the resonant circuit changes.

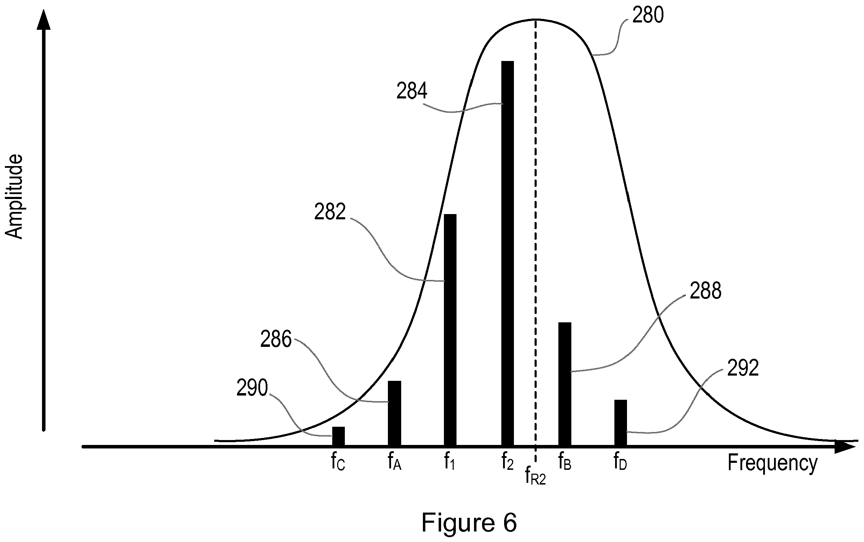

Figure 6 shows the effect of this operation, in a situation where the resonance curve of the resonant circuit 210 is illustrated by the line 280.

The resonant frequency $f_{R2}$ still depends on the inductance of the inductor 212 and the capacitance of the capacitor 214, but the change in the capacitance due to the pressure in the body means that the resonant frequency of the resonant circuit has changed.

Figure 6 shows the situation when, as illustrated in Figure 5, the driving circuit 204 is configured to generate a driving signal comprising a first component at a first frequency $f_1$ and a second component at a second frequency $f_2$. The new resonant frequency $f_{R2}$ is higher than the second frequency $f_2$.

Thus, Figure 6 shows that the measurement circuit 202 produces a response 282 to the first component of the driving signal at the first frequency $f_1$ and produces a response 284 to the second component of the driving signal at the second frequency $f_2$. Because the new resonant frequency $f_{R2}$ is higher than the second frequency $f_2$, as shown by way of illustration in Figure 5, the response 284 is larger than the response 282.

As before, the effect of including the non-linear element 216 in the measurement circuit 202 is that its response also includes intermodulation components, and it is the odd-order intermodulation components that are relevant.

Figure 6 illustrates the third-order intermodulation components. Specifically, Figure 6 illustrates a first third-order intermodulation component 286 at the frequency $f_A$ (= $2.f_1 - f_2$) and a second third-order intermodulation component 288 at the frequency $f_B$ (= $2.f_2 - f_1$).

Since the new resonant frequency $f_{R2}$ is higher than the second frequency $f_2$, as shown by way of illustration in Figure 6, the second third-order intermodulation component 288 has a larger magnitude than the first third-order intermodulation component 286.

If the first frequency $f_1$ and the second frequency $f_2$ are sufficiently close together, the frequency $f_A$ of the first third-order intermodulation component 286 and the frequency $f_B$ of the second third-order intermodulation component 288 are both within (or close to) the effective bandwidth of the resonant circuit 210.

Figure 6 also illustrates the fifth-order intermodulation components. Specifically, Figure 6 illustrates a first fifth-order intermodulation component 290 at the frequency $f_C$ (= $3.f_1 - 2.f_2$) and a second fifth-order intermodulation component 292 at the frequency $f_D$ (= $3.f_2 - 2.f_1$).

Since the new resonant frequency $f_{R2}$ is higher than the second frequency $f_2$, as shown by way of illustration in Figure 6, the second fifth-order intermodulation component 292 has a larger magnitude than the first fifth-order intermodulation component 290.

If the first frequency $f_1$ and the second frequency $f_2$ are sufficiently close together, the frequency $f_C$ of the first fifth-order intermodulation component 290 and the frequency $f_D$ of the second fifth-order intermodulation component 292 are both within (or close to) the effective bandwidth of the resonant circuit 210.

The response of the measurement circuit 202 to the driving signals generated by the driving circuit 204 is measured by the frequency analyzer circuit 206.

Specifically, the frequency analyzer circuit 206 comprises a spectrum analyzer, which measures the amplitudes of signal components at different frequencies.

The frequency analyzer circuit 206 can therefore distinguish the intermodulation products from the components of the drive signal itself.

Based on the measured amplitudes of the signal components at the different frequencies, the frequency analyzer circuit 206 is able to determine the shift in the resonant frequency of the measurement circuit 202, and hence is able to determine the change in the value of the electrical property of the variable component of the resonant circuit. That is, when the resonant circuit contains a capacitor, whose capacitance depends on the pressure or other parameter in the body, or an inductor whose inductance depends on the pressure or other parameter in the body, the shift in the resonant frequency of the measurement circuit can be used to determine the value of the variable capacitance or inductance (as the case may be) and hence can be used to determine the value of the pressure or other parameter in the body.

For example, the shift in the resonant frequency can be determined by examining the ratio of the amplitudes of the intermodulation products.

More specifically, if the shape of the resonance curve 280 shown in Figure 6 is known, then the ratio of the amplitude of the second third-order intermodulation component 288 to the amplitude of the first third-order intermodulation component 286 will indicate the shift in the resonant frequency.

Similarly, the ratio of the amplitude of the second fifth-order intermodulation component 292 to the amplitude of the first fifth-order intermodulation component 290 will indicate the shift in the resonant frequency.

Thus, either the third-order intermodulation components, or the fifth-order intermodulation components, or similarly any higher odd-order intermodulation components that are large enough to be detected, can be used. Amplitude ratios of more than one of the odd-order intermodulation components can be found, and combined (for example, averaged) to obtain a measurement result.

This shift in the resonant frequency in turn will indicate the shift in the variable property of the variable component of the resonant circuit. Finally, knowledge of the characteristics of this variable component will allow a determination of the property to be measured in the body of the patient.

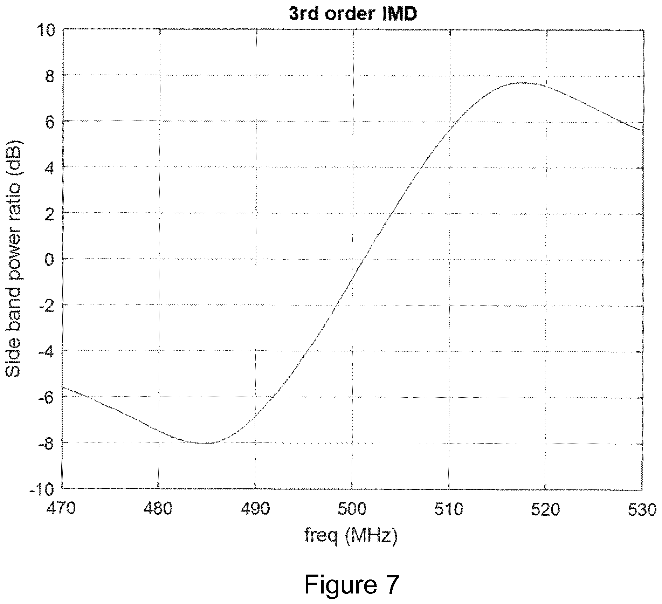

Figure 7 illustrates an example where the baseline resonant frequency is about 500 MHz. Specifically, Figure 7 illustrates the relationship between the ratio of the amplitudes of the two third-order intermodulation products and the resonant frequency. Thus, when the resonant frequency is midway between the frequencies of the driving signals generated by the driving circuit 204 (and assuming that the two driving signals generated by the driving circuit 204 have equal amplitudes), the response of the measurement circuit contains components at these two frequencies that are of equal amplitude, and hence the power ratio is 0dB. In the example illustrated in Figure 7, this occurs when the resonant frequency is about 501 MHz.

As the resonant frequency increases, the power ratio (that is the ratio of the power of the second third-order intermodulation component 288 to the power of the first third-order intermodulation component 286, as shown in Figure 6) increases. For example, when the resonant frequency is 510 MHz, the power ratio is about 6dB. Similarly, as the resonant frequency decreases, the power ratio (that is the ratio of the power of the second third-order intermodulation component 288 to the power of the first third-order intermodulation component 286, as shown in Figure 6) decreases. For example, when the resonant frequency is 490 MHz, the power ratio is about -7dB.

Thus, by measuring the power ratio, it is possible to determine the resonant frequency (provided that this varies within the range from about 485-515MHz, in this example), and hence it is possible to determine the value of the electrical property of the component of the resonant circuit that depends on the pressure or other parameter in the body. Based on knowledge of the characteristic of this component, it is then possible to determine the value of the pressure or other parameter in the body.

Similarly, the shift in the resonant frequency can be determined by examining the ratio of the amplitudes of the fifth-order intermodulation products, or any suitable higher-order intermodulation products.

The use of a ratio measurement as described here is robust against disturbances such as movements of the patient, effects due to changes in temperature, and the aging of components of the device.

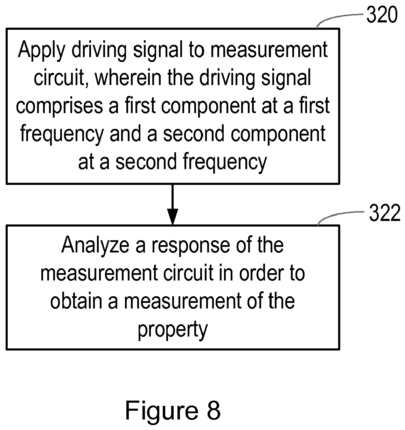

Figure 8 is a flow chart, illustrating a method of measuring a property in a body.

In step 320, a driving signal is applied to a measurement circuit. The driving signal comprises a first component at a first frequency and a second component at a second frequency. As described above, the measurement circuit comprises a resonant circuit comprising a first element and a second element, and at least one of the first element and the second element is configured such that a property thereof is dependent on the property in the body. The measurement circuit also comprises a non-linear element arranged to generate intermodulation products when the circuit is driven by an input signal comprising a first component at a first frequency and a second component at a second frequency.

In step 322, a response of the measurement circuit is analyzed in order to obtain a measurement of the property in the body.

The examples of methods described above use two different excitation frequencies to measure a variable electrical property of a measurement circuit. However, the invention is not limited to electrical fields, but can also be extended to ultrasonic or optical fields (using a non-linear element). For example, the measurement circuit can include an ultrasonic resonator with a mechanical non-linearity, which is sensitive to a property to be measured in the body, and the driving circuit can then apply ultrasonic signals, which will result in ultrasonic sidebands. As described above, the magnitudes of the intermodulation components can be used to detect the change in the resonant frequency of the resonant circuit. Also as described above, this can then be used to determine the change in the property of the resonator, and hence the change in the property to be measured in the body.

As another example, the measurement circuit can include an optical resonator with an optical non-linear element, which can generate intermodulation products when, for example, two laser beams at different wavelengths are applied to it. As described above, the magnitudes of the intermodulation components can be used to detect the change in the resonant frequency of the resonant circuit. Also as described above, this can then be used to determine the change in the property of the resonator, and hence the change in the property to be measured in the body can cause new light wave.

There is therefore provided a method and a circuit for measuring a property in a body.

The invention is defined by appended claims 1-14.

Any reference signs in the claims should not be construed as limiting the scope.

**Claims**

1. A measurement system for measuring a property in a body, the measurement system comprising:

   a measurement circuit for measuring a property in a body, the measurement circuit comprising:

   a resonant circuit comprising a first element and a second element, wherein at least one of the first element and the second element is configured such that a property thereof is dependent on the property in the body; and a non-linear element arranged to generate intermodulation products when the circuit is driven by an input signal comprising a first component at a first frequency and a second component at a second frequency;

   a driving circuit for providing a driving signal to the measurement circuit, wherein the driving signal comprises a first component at a first frequency and a second component at a second frequency; and
   an analyzer circuit for analyzing a response of the measurement circuit in order to obtain a measurement of the property, wherein the analyzer circuit is configured to measure a ratio of the amplitudes of odd order intermodulation products resulting from the first and second components of the driving signal.

2. A measurement system according to claim 1, wherein at least one of the first element and the second element is configured such that an electrical property thereof is dependent on the property in the body.

3. A measurement system according to claim 2, wherein the first element is an inductance and the second element is a capacitor.

4. A measurement system according to claim 2 or 3, wherein the first element and the second element are arranged in parallel.

5. A measurement system according to any of the claims 2 to 4, wherein the non-linear element comprises two antiparallel diodes.

6. A measurement system according to claim 5, wherein the first element is an inductance and the second element is a capacitor, wherein the inductance and the capacitor are arranged in parallel, and wherein the two antiparallel diodes are connected in parallel with the inductance and the capacitor.

7. A measurement system according to any of the claims 2 to 6, wherein an electrical property of at least one of the first element and the second element is dependent on pressure in the body.

8. A measurement system according to any preceding claim, wherein the driving circuit is arranged to provide the driving signal comprising the first component at the first frequency and the second component at the second frequency, and wherein the first frequency is above a baseline resonant frequency of the resonant circuit and the second frequency is below the baseline resonant frequency of the resonant circuit.

9. A measurement system according to any preceding claim, wherein the driving circuit is arranged to provide the driving signal comprising the first component at the first frequency and the second component at the second frequency; and wherein the first and the second frequency fall within the bandwidth of the resonant circuit.

10. A measurement system according to any preceding claim, wherein the analyzer circuit comprises a frequency analyzer.

11. A measurement system according to any preceding claim, further comprising a wire connection connecting the driving circuit and the measurement circuit.

12. A measurement system according to any of claims 1 to 10, further comprising a transmitter arranged to wirelessly transmit the driving signal from the driving circuit to the measurement circuit.

13. A measurement system according to any preceding claim, wherein the system is arranged to wirelessly transmit the response of the measurement circuit to the analyzer circuit.

14. A method of measuring a property in a body, the method comprising:

applying a driving signal to a measurement circuit, wherein the driving signal comprises a first component at a first frequency and a second component at a second frequency, and wherein the measurement circuit comprises:

a resonant circuit comprising a first element and a second element, wherein at least one of the first element and the second element is configured such that a property thereof is dependent on the property in the body; and a non-linear element arranged to generate intermodulation products when the circuit is driven by an input signal comprising a first component at a first frequency and a second component at a second frequency; and

analyzing a response of the measurement circuit in order to obtain a measurement of the property, wherein analyzing the response of the measurement circuit comprises measuring a ratio of the amplitudes of odd order intermodulation products resulting from the first and second components of the driving signal.

**Patentansprüche**

1. Ein Messsystem zum Messen einer Eigenschaft in einem Körper, wobei das Messsystem Folgendes umfasst:

einen Messkreis zum Messen einer Eigenschaft in einem Körper, wobei der Messkreis Folgendes umfasst:

einen Schwingkreis mit einem ersten und einem zweiten Element, wobei mindestens das erste oder zweite Element so konfiguriert ist, dass eines seiner Eigenschaften von der Eigenschaft des Körpers abhängig ist; und ein nicht lineares Element zum Generieren von Intermodulationsprodukten, wenn der Kreis von einem Eingangssignal angetrieben wird, das eine erste Komponente mit einer ersten Frequenz und eine zweite Komponente mit einer zweiten Frequenz umfasst;

ein Antriebskreis zum Bereitstellen eines Antriebssignals für den Messkreis, wobei das Antriebssignal über eine erste Komponente mit einer ersten Frequenz und eine zweite Komponente mit einer zweiten Frequenz verfügt; und einen Analysekreis zum Analysieren einer Reaktion auf den Messkreis, um eine Messung der Eigenschaft abzurufen, wobei der Analysekreis ein Verhältnis der Amplituden von Intermodulationsprodukten mit ungerader Reihenfolge misst, das sich aus den ersten und zweiten Komponenten des Antriebssignals ergibt.

2. Ein Messsystem gemäß Anspruch 1, wobei mindestens das erste oder zweite Element so konfiguriert ist, dass eines seiner Eigenschaften von einer elektrischen Eigenschaft des Körpers abhängig ist.

3. Ein Messsystem gemäß Anspruch 2, wobei es sich beim ersten Element um eine Induktanz und beim zweiten Element um einen Kondensator handelt.

4. Ein Messsystem gemäß Anspruch 2 oder 3, wobei das erste und das zweite Element parallel angeordnet sind.

5. Ein Messsystem gemäß einer der Ansprüche 2 bis 4, wobei das nicht lineare Element zwei antiparallele Dioden umfasst.

6. Ein Messsystem gemäß Anspruch 5, wobei es sich beim ersten Element um eine Induktanz und beim zweiten Element um einen Kondensator handelt, und wobei die Induktanz und der Kondensator parallel angeordnet und die beiden antiparallelen Dioden parallel an der Induktanz und am Kondensator angeschlossen sind.

7. Ein Messsystem gemäß einer der Ansprüche 2 bis 6, wobei eine elektrische Eigenschaft des mindestens ersten oder zweiten Elements vom Druck im Körper abhängig ist.

8. Ein Messsystem gemäß einer der vorherigen Ansprüche, wobei der Antriebskreis das Antriebssignal mit der ersten Komponente mit der ersten Frequenz und der zweiten Komponente mit der zweiten Frequenz bereitstellt, und wobei die erste Frequenz über einer Grundschwingfrequenz des Schwingkreises liegt, während die zweite Frequenz unter der Grundschwingfrequenz des Schwingkreises liegt.

9. Ein Messsystem gemäß einer der vorherigen Ansprüche, wobei der Antriebskreis das Antriebssignal mit der ersten Komponente mit der ersten Frequenz und der zweiten Komponente mit der zweiten Frequenz bereitstellt; und wobei sich die erste und die zweite Frequenz innerhalb der Bandbreite des Schwingkreises bewegen.

10. Ein Messsystem gemäß einer der vorherigen Ansprüche, wobei der Analysekreis eine Frequenzanalyse umfasst.

11. Ein Messsystem gemäß einer der vorherigen Ansprüche, das zudem eine Drahtverbindung zwischen dem Antriebs- und dem Messkreis umfasst.

12. Ein Messsystem gemäß einer der Ansprüche 1 bis 10, das zudem einen Sender für das drahtlose Übertragen des Antriebssignals vom Antriebs- zum Messkreis umfasst.

13. Ein Messsystem gemäß einer der vorherigen Ansprüche, wobei das System die Reaktion des Messkreises drahtlos an den Analysekreis überträgt.

14. Eine Methode zum Messen einer Eigenschaft in einem Körper, wobei die Methode folgende Schritte umfasst: Übernehmen eines Antriebssignals für den Messkreis, wobei das Antriebssignal über eine erste Komponente mit einer ersten Frequenz und eine zweite Komponente mit einer zweiten Frequenz verfügt, und wobei der Messkreis Folgendes umfasst:

einen Schwingkreis mit einem ersten und einem zweiten Element, wobei mindestens das erste oder zweite Element so konfiguriert ist, dass eines seiner Eigenschaften von der Eigenschaft des Körpers abhängig ist; und
ein nicht lineares Element zum Generieren von Intermodulationsprodukten, wenn der Kreis von einem Eingangssignal angetrieben wird, das eine erste Komponente mit einer ersten Frequenz und eine zweite Komponente mit einer zweiten Frequenz umfasst; und
Analysieren einer Reaktion auf den Messkreis, um eine Messung der Eigenschaft abzurufen, wobei das Analysieren der Reaktion des Messkreises das Messen eines Verhältnisses der Amplituden von Intermodulationsprodukten mit ungerader Reihenfolge umfasst, das sich aus den ersten und zweiten Komponenten des Antriebssignals ergibt.

**Revendications**

1. Système de mesure permettant de mesurer une propriété dans un corps, ledit système de mesure comprenant :

un circuit de mesure pour mesurer une propriété dans un corps, ledit circuit de mesure comprenant :

un circuit résonnant comprenant un premier élément et un second élément, dans lequel le premier élément et/ou le second élément est conçu de telle sorte qu'une propriété de celui-ci dépend de la propriété dans le corps ; et
un élément non linéaire conçu pour générer des produits d'intermodulation lorsque le circuit est commandé par un signal d'entrée comprenant un premier composant à une première fréquence et un second composant à une seconde fréquence ;

un circuit d'attaque pour fournir un signal d'attaque au circuit de mesure, dans lequel le signal d'attaque comprend un premier composant à une première fréquence et un second composant à une seconde fréquence ; et
un circuit analyseur pour analyser une réponse du circuit de mesure pour obtenir une mesure de la propriété, dans lequel le circuit analyseur est conçu pour mesurer un rapport des amplitudes des produits d'intermodulation d'ordre impair résultant des premier et second composants du signal d'attaque.

2. Système de mesure selon la revendication 1, dans lequel le premier élément et/ou le second élément est conçu de telle sorte qu'une propriété électrique de celui-ci dépend de la propriété dans le corps.

3. Système de mesure selon la revendication 2, dans lequel le premier élément est une inductance et le second élément est un condensateur.

4. Système de mesure selon la revendication 2 ou 3, dans lequel le premier élément et le second élément sont disposés en parallèle.

5. Système de mesure selon l'une quelconque des revendications 2 à 4, dans lequel l'élément non linéaire comprend deux diodes antiparallèles.

6. Système de mesure selon la revendication 5, dans lequel le premier élément est une inductance et le second élément est un condensateur, dans lequel l'inductance et le condensateur sont disposés en parallèle, et dans lequel les deux diodes antiparallèles sont connectées en parallèle avec l'inductance et le condensateur.

7. Système de mesure selon l'une quelconque des revendications 2 à 6, dans lequel une propriété électrique du premier élément et/ou du second élément dépend de la pression dans le corps.

8. Système de mesure selon l'une quelconque des revendications précédentes, dans lequel le circuit d'attaque est conçu pour fournir le signal d'attaque comprenant le premier composant à la première fréquence et le second composant à la seconde fréquence,

et dans lequel la première fréquence est supérieure à une fréquence de résonance de base du circuit résonnant et la seconde fréquence est inférieure à la fréquence de résonance de base du circuit résonnant.

9. Système de mesure selon l'une quelconque des revendications précédentes, dans lequel le circuit d'attaque est conçu pour fournir le signal d'attaque comprenant le premier composant à la première fréquence et le second composant à la seconde fréquence ; et
dans lequel la première et la seconde fréquence se situent dans la bande passante du circuit résonnant.

10. Système de mesure selon l'une quelconque des revendications précédentes, dans lequel le circuit analyseur comprend un analyseur de fréquence.

11. Système de mesure selon l'une quelconque des revendications précédentes, comprenant en outre une connexion filaire connectant le circuit d'attaque et le circuit de mesure.

12. Système de mesure selon l'une quelconque des revendications 1 à 10, comprenant en outre un émetteur conçu pour émettre sans fil le signal d'attaque du circuit d'attaque au circuit de mesure.

13. Système de mesure selon l'une quelconque des revendications précédentes, dans lequel ledit système est conçu pour transmettre sans fil la réponse du circuit de mesure au circuit analyseur.

14. Procédé de mesure d'une propriété dans un corps, ledit procédé comprenant : l'application d'un signal d'attaque à un circuit de mesure, dans lequel le signal d'attaque comprend un premier composant à une première fréquence et un second composant à une seconde fréquence, et dans lequel le circuit de mesure comprend :

　　　un circuit résonnant comprenant un premier élément et un second élément, dans lequel le premier élément et/ou le second élément est conçu de telle sorte qu'une propriété de celui-ci dépend de la propriété dans le corps ; et
　　　un élément non linéaire conçu pour générer des produits d'intermodulation lorsque le circuit est commandé par un signal d'entrée comprenant un premier composant à une première fréquence et un second composant à une seconde fréquence ; et
　　　l'analyse d'une réponse du circuit de mesure pour obtenir une mesure de la propriété, dans lequel l'analyse de la réponse du circuit de mesure comprend la mesure d'un rapport des amplitudes des produits d'intermodulation d'ordre impair résultant des premier et second composants du signal d'attaque.

Figure 1

Figure 2

Figure 3

Figure 4

Figure 5

Figure 6

EP 3 592 209 B1

3rd order IMD

Side band power ratio (dB)

freq (MHz)

Figure 7

320

Apply driving signal to measurement
circuit, wherein the driving signal
comprises a first component at a first
frequency and a second component
at a second frequency

322

Analyze a response of the
measurement circuit in order to
obtain a measurement of the
property

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012109039 A
- WO 2014164734 A
- WO 2015099845 A
- WO 2004014456 A
- WO 2017013224 A
- WO 2016207041 A
- US 20130060139 A1